# EUROPEAN PATENT SPECIFICATION

(11) **EP 4 183 339 B1**
(45) Date of publication and mention of the grant of the patent: **01.04.2026**
(21) Application number: 21843300.1
(22) Date of filing: 31.05.2021
(51) Int. Cl.: A61B 5/145

(54) **HIGH-RELIABILITY ANALYTE DETECTION DEVICE**
HOCHZUVERLÄSSIGE ANALYTDETEKTIONSVORRICHTUNG
DISPOSITIF DE DÉTECTION D'ANALYTE DE FIABILITÉ ÉLEVÉE

(30) Priority: 15.07.2020 WO PCT/CN2020/102017; 03.08.2020 WO PCT/CN2020/106518; 03.08.2020 WO PCT/CN2020/106522
(43) Date of publication of application: 24.05.2023
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/097173
(87) International publication number: WO 2022/012187

(56) References cited:
- WO-A1-2020/027424
- CN-A- 106 470 593
- CN-A- 106 470 593
- CN-A- 109 998 560
- CN-A- 109 998 560
- CN-A- 110 584 676
- CN-A- 112 386 251
- CN-U- 207 898 483
- US-A1- 2014 121 989
- US-A1- 2018 296 146

## Description

### TECHNICAL FIELD

The invention mainly relates to the field of medical devices, in particular to a high reliability analyte detection device.

### BACKGROUND

The pancreas in a normal human body can automatically monitor the blood glucose level and automatically secrete required amount of insulin/glucagon. In the body of a type 1 diabetes patient, the pancreas does not function properly and cannot produce enough insulin for the body. Therefore, type 1 diabetes is a metabolic disease caused by abnormal pancreatic function, and diabetes is a lifelong disease. At present, there is no cure for diabetes with medical technology. The onset and development of diabetes and its complications can only be controlled by stabilizing blood glucose.

Diabetics need to have their blood glucose measured before they inject insulin into the body. At present, most of the testing methods can continuously measure blood glucose level and transmit the data to a remote device in real time for the user to view. This method is called Continuous Glucose Monitoring (CGM).

However, the current detection device is complex and not compact, and the overall volume of the detection device is large. When the user's stretching, movement or dressing are affected, the detection device is easy to be touched, or the conductive parts are damaged when the components are disassembled and replaced, which makes the detection device lose effective electrical connection and interrupt the detection, leading to the loss of detection data, bringing safety risks to users

CN 109 998 560 A describes an analyte detection device with two conductive rubbers that can be inserted in holes of a silicone seat for connecting the sensor with the transmitter.

Therefore, the prior art urgently needs a highly reliable analyte detection device with good electrical connection performance and easy disassembly.

### BRIEF SUMMARY OF THE INVENTION

The embodiment of the invention discloses a highly reliable analyte detection device. The signal output end contacts with the elastic member, which improves the reliability of the internal electrical connection of the detection device while reducing the number of structures inside the device. When the transmitter is disassembled or replaced, the conductive part will not be damaged, so as to avoid data loss due to loss of effective electrical connection and enhance the user experience.

The invention discloses a high reliability analyte detection device, which comprises a transmitter which is provided with at least one first clamp part and at least two mutually insulated first electrical connection areas. The bottom shell is provided with the second clamp part corresponding to the first clamp part. The first clamp part and the second clamp part are clamped to each other, and the transmitter is assembled on the bottom shell. The bottom shell comprises a fixed part and a forced part. When separating the bottom shell and the transmitter, the fixed part is fixed, and the force is applied to the forced part in one direction, the bottom shell fails. At least one pair of first and second clamp parts that are clamped to each other are separated from each other, and further separating the bottom shell from the transmitter. The sensor is assembled on the bottom shell. The sensor comprises a signal output end and a detection end. The signal output end is provided with at least two second electrical connection areas corresponding to the first electrical connection area and mutually insulated. The first electrical connection area is electrically connected with the corresponding second electrical connection area. An elastic member, wherein the signal output end contacts the elastic member. And battery, connected to the transmitter, for supplying power to the transmitter.

According to one aspect of the invention, the first electrical connection area is a metal contact.

According to one aspect of the invention, the signal output end is bent or bent towards the bottom surface of the bottom shell.

According to one aspect of the invention, the signal output end is arranged on the top of the elastic member, and the first electrical connection area is directly connected with the corresponding second electrical connection area.

According to one aspect of the invention, the elastic member comprises at least two conductive areas and at least one insulating area, an insulating area is arranged between two adjacent conductive areas, at least two first electrical connection areas are indirectly electrically connected with the corresponding second electrical connection area through different conductive areas, and different first electrical connection areas or different second electrical connection areas are indirectly electrically connected with different conductive areas.

According to one aspect of the invention, the conductive area and the insulating area pass through the elastic member in the longitudinal direction respectively.

According to one aspect of the invention, the signal output end is embedded in the elastic member or arranged at the bottom of the elastic member.

According to one aspect of the invention, different first electrical connection areas are set on different parts of the signal output end, and different parts of the signal output end are independent of each other.

According to one aspect of the invention, the signal output end is embedded in the elastic member, and the height of the embedded position of the signal output end of different parts in the elastic member is not exactly the same.

According to one aspect of the invention, the signal output end of each part is embedded in the elastic member, or is arranged at the bottom of the elastic member, or is arranged at the top of the elastic member.

According to one aspect of the invention, the number of the first electrical connection area and the second electrical connection area is three.

According to one aspect of the invention, the part of the bottom shell that is equipped with the transmitter is a forced part.

According to one aspect of the invention, the side of the bottom shell is provided with an outward convex part, and the convex part is the forced part.

According to one aspect of the invention, the battery is arranged in the bottom shell, and at least one connection hole is arranged in the bottom shell. Through the connection hole, the transmitter is electrically connected with the two poles of the battery, and the battery part is the forced part.

According to one aspect of the invention, a sealing ring is arranged around the connection hole to seal the electrical connection position. When the force is applied to the forced part, the sealing ring provides the elastic force to promote the separation of the bottom shell and the transmitter.

Compared with the prior art, the technical scheme of the invention has the following advantages:
In the high reliability analyte detection device disclosed by the invention, the sensor is assembled on the bottom shell, the sensor comprises a signal output end and a detection end, the signal output end is bent or bent to the bottom surface of the bottom shell, and the signal output end is in contact with the elastic member. When the first electrical connection area and the second electrical connection area are electrically connected, according to the position set in the first electrical connection area, the same elastic member plays the role of conducting, carrying or buffering, improving the reliability of electrical connection, while reducing the number of structures inside the device. The transmitter can be disassembled by bending the forced part, which is more convenient for users to disassemble and replace the transmitter. The electrical connection area is not in the bending area, which will not damage the conductive parts, avoid the loss of detection data, improve the reliability of analyte detection devices, and enhance the user experience.

Further, the elastic member comprises at least two conductive areas and at least one insulating area. An insulating area is arranged between two adjacent conductive areas. At least two first electrical connection areas are indirectly electrically connected with the corresponding second electrical connection area through different conductive areas. Different first electrical connection areas or different second electrical connection areas are indirectly electrically connected with different conductive areas. One elastic member plays the role of conducting and insulating at the same time, which not only reduces the number of internal structures of the detection device, but also acts as a buffer, enhancing the stability of the electrical connection between the transmitter and the sensor.

Further, different first electrical connection areas are arranged on different parts of the signal output end, and different parts of the signal output end are independent of each other and do not interfere with each other. In the actual manufacturing process, the thickness of each first electrical connection area will be different. When the transmitter is connected with the sensor, this mutually independent and non-interference first electrical connection area can weaken or eliminate the influence of poor contact caused by the above thickness difference, and improve the stability of electrical connection between elastic members, the first electrical connection area and the second electrical connection area.

### BRIEF DESCRIPTION OF THE DRAWINGS

Fig. 1 is the three-dimensional structure diagram of the bottom shell according to an embodiment of the invention.
Fig. 2 is the assembly diagram of the sensor and the bottom shell according to an embodiment of the invention.
Fig. 3 is the three-dimensional structure diagram of a transmitter according to an embodiment of the invention.
Figs. 4a to 4b are the structural diagrams of the elastic member, the first electrical connection area and the second electrical connection area according to an embodiment of the invention, Fig. 4a is a top view, and Fig. 4b is a side view of the structure of Fig. 4a.
Fig. 4c is the top structure diagram of the elastic member and the first electrical connection area according to another embodiment of the invention.
Figs. 4d and 4e are the top structure diagrams of the elastic member, the first electrical connection area and the second electrical connection area according to different embodiments of the invention.
Fig. 5 is the structural diagram of the elastic member, the first electrical connection area and the second electrical connection area according to another embodiment of the invention.
Figs. 6a and 6b are structural diagrams of the second electrical connection area and the electrical connection position of the elastic member according to different embodiments of the invention.
Figs. 7a and 7b are the structural diagrams of the elastic member electrically connected with the first electrical connection area and the second electrical connection area respectively according to another embodiment of the invention, and Fig. 7b is the sectional view obtained along the section line A-A' in Fig. 7a.
Figs. 8a and 8b are the structural diagrams of the elastic member electrically connected with the first electrical connection area and the second electrical connection area respectively according to another embodiment of the invention, and Fig. 8b is the sectional view obtained along the section line B-B' in Fig. 8a.
Fig. 9a is the schematic diagram of the three-dimensional structure of the second electrical connection area according to another embodiment of the invention.
Fig. 9b is the schematic diagram of the three-dimensional structure of the elastic member matched with the second electrical connection area in Fig. 9a and the signal output end.
Fig. 10 is the structural diagram showing that the signal output end is arranged on the top of the elastic member according to another embodiment of the invention.
Fig. 11 is the structural diagram showing that the signal output ends of different parts are set at different positions of the elastic member according to another embodiment of the invention.

### DETAILED DESCRIPTION

As mentioned above, the detection data of the body fluid parameter detection device in the prior art is easy to lose, and the user experience is poor, which brings inconvenience to the patient's life.

Through research, it is found that the causes of the above problems are: on the one hand, multiple conductive parts need to be set between the transmitter and the sensor of the existing detection device, and additional insulating parts are also set to separate the adjacent conductive parts. The internal structure of the detection device is complex, the structure is not compact enough, and the electrical connection performance is poor. On the other hand, users are prone to damage the conductive parts when disassembling the transmitter, and the electrical connection is unstable, resulting in data loss.

In order to solve this problem, the invention provides a highly reliable analyte detection device, wherein the signal output end contacts with the same elastic member, the transmitter can be disassembled by bending the forced part, which does not affect the conductive part, avoids damage to the conductive part, improves the reliability of the electrical connection of the detection device, avoids data loss, and enhances the user experience.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

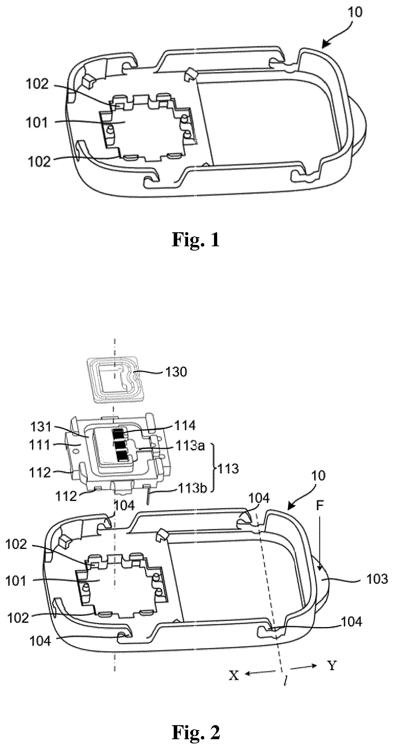

Fig. 1 shows the three-dimensional structure of the bottom shell 10 in the embodiment of the invention. Fig. 2 is the assembly diagram of the sensor 113 and the bottom shell 10 in the embodiment of the invention. Detailed description will be made with reference to Fig. 1 and Fig. 2.

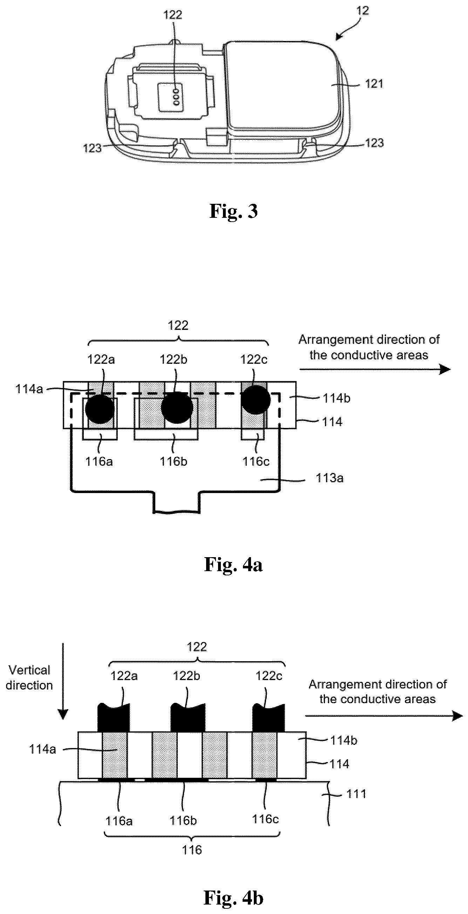

The bottom shell 10 is used for assembling the sensor 113 and the transmitter 12. In the embodiment of the invention, the bottom surface of the bottom shell 10 is provided with an assembly hole 101 for assisting in the installation of the sensor 113, and the first clamp structure 102 is arranged around the assembly hole 101 to assist in the installation of the sensor 113 on the bottom shell 10. In combination with Figs. 2 and 3, the side wall of the bottom shell 10 is also provided with a second clamp part 104 for fixing the transmitter 12. Correspondingly, the transmitter 12 is provided with a first clamp part 123 that can engage with the second clamp part 104.

In the embodiment of the invention, the number of the second clamp parts 104 is two, and the two second clamp parts 104 correspond to the side walls of the bottom shell 10.

In other embodiments of the invention, the number of the second clamp parts 104 is four, and the four second clamp parts 104 are correspondingly arranged on the opposite side walls of the bottom shell 10, two on each side.

In other embodiments of the invention, the number of the second clamp parts 104 is six, and the six second clamp parts 104 correspond to the side walls of the bottom shell 10, with two on each side.

In the embodiment of the invention, bending the forced part can make the bottom shell 10 fail. The failure mode of the bottom shell comprises one or more combinations of the bottom plate fracture, the bottom shell fracture, the second clamp part fracture, and the bottom shell deformation of the bottom shell 10. In a word, after the bottom shell fails, the first clamp part 123 and the second clamp part 104 are separated from the clamp connection, and the transmitter 12 can be separated from the bottom shell 10. Because of the electrical connection parts, the second electrical connection area 122 of the transmitter 12 is not located in the forced part, The user will not damage the electrical connection parts when disassembling the transmitter 12. After replacing the bottom shell 10, install the transmitter 12 on the new bottom shell, which will not affect the electrical connection between the second connection area 122 and the first electrical connection area 116 of the new bottom shell, and ensure the stability of the electrical connection.

In the embodiment of the invention, the fixed part and the forced part are relative concepts. According to the structural design of the bottom shell 10 and the transmitter 12, the positions of the fixed part and the forced part can be selected differently.

In other embodiments of the invention, the connecting line *l* of the two second clamp parts 104 divides the bottom shell 10 into X side and Y side. The Y side is provided with a forced part, and the X side is provided with a fixed part.

Therefore, in the embodiment of the invention, the process of separating the bottom shell 10 from the transmitter 12 is as follows: fix the fixed part on the X side with a finger, and apply force F to the forced part on the Y side with another finger in one direction to make the second clamp part 104 invalid, and then separate the second clamp part 104 from the first clamp part 123, so that the transmitter 12 is separated from the bottom shell 10.

In the embodiment of the invention, the forced part is a convex part 103 facing outward from the side of the bottom shell. The shape, size and number of the convex part 103 are not limited. Preferably, the convex part 103 is semicircular arc, which is convenient for users to press with their fingers, saves space, and ensures a small and compact bottom shell structure.

In other embodiments of the invention, the bottom shell 10 can also be of other shapes, as long as the conditions for installing the transmitter 12 and the sensor 113 on the bottom shell 10 can be met, there is no specific restriction here.

The sensor 113 can be assembled on the bottom shell 10 in various ways, and there are no specific restrictions here. Specifically, in the embodiment of the invention, the bottom shell 10 comprises a sensor base 111. The sensor 113 is mounted to the bottom shell 10 through the sensor base 111. A second clamp structure 112 is arranged around the sensor base 111. The second clamp structure 112 is clamped with the first clamp structure 102 to install the sensor base 111 in the assembly hole 101, and then the sensor 113 is assembled on the bottom shell 10.

In another embodiment of the invention, after the sensor 113 is installed on the bottom shell 10, the auxiliary mounting structure of the sensor 113 is removed, and the sensor 113 is not loaded by the sensor base 111 or other components, but is separately installed on the bottom shell 10.

In other embodiments of the invention, the sensor 113 can also be assembled to the bottom shell 10 in other ways, without specific restrictions.

It should be noted that in the embodiment of the invention, the sensor base 111 is also provided with a sealing ring 130 and a groove 131 for placing the sealing ring 130.

Referring further to Fig. 2, the sensor 113 comprises a signal output end 113a and a detection end 113b. The signal output end 113a needs to be electrically connected with the second electrical connection area 122 of the transmitter 12 to transmit the detection signal to the transmitter 12. The detection end 113b is used to pierce the subcutaneous tissue of human body to detect the parameter information of body fluid analyte.

The signal output end 113a is provided with a first electrical connection area 116 which is mutually insulated. Conventionally, the sensor 113 is also provided with electrodes and/or electrode wires (not shown here or below) for detecting analyte parameter information. The detection signal of the electrode needs to be exported through the first electrical connection area 116.

It should be noted that the embodiment of the invention does not limit the setting mode of the first electrical connection area 116 on the signal output end 113a. For example, the first electrical connection area 116 can be set on the surface of the signal output end 113a or embedded in the signal output end 113a.

Generally, the sensor 113 is provided with at least two detection electrodes, that is, at least including a working electrode and a counter electrode. Therefore, in the implementation of the invention, at least two first electrical connection areas 116 are arranged on the surface of the signal output end 113a for electrical connection with different electrodes. Specifically, in the embodiment of the invention, the sensor 113 is a three-electrode system. Therefore, the number of the first electrical connection area 116 is three.

As shown in Fig. 2, in the embodiment of the invention, the signal output end 113a bends or bends to the bottom surface of the bottom shell 10. The signal output end 113a is fitted with the surface of the sensor base 111 or embedded in the sensor base 111. This design reduces the height of the sensor 113 protruding from the bottom shell 10 and reduces the thickness of the detection device.

In other embodiments of the invention, the sensor 113 can also be of other shapes or forms (such as non-bending), which is not specifically limited here.

Fig. 3 shows the three-dimensional structure of the transmitter 12 in the embodiment of the invention.

The transmitter 12 is provided with a second electrical connection area 122 which is mutually insulated. The second electrical connection area 122 is used for electrical connection with the first electrical connection area 116 to receive electrical signals from the sensor 113. Therefore, the second electrical connection area 122 corresponds to the first electrical connection area 116.

Here, correspondence means that the two are equal in number and their positions are basically corresponding. Obviously, in the embodiment of the invention, the number of the second electrical connection area 122 is three, so as to adapt to the three-electrode system of the sensor 113.

In the embodiment of the invention, the second electrical connection area 122 is exposed and protrudes from the shell 121 of the transmitter. Specifically, in the embodiment of the invention, the second electrical connection area 122 is a metal contact. The smaller metal contact makes the internal structure of the detection device more compact, and the volume of the detection device will be further reduced.

In the embodiment of the invention, the battery (not shown in the figure) is arranged in the shell 121 of the transmitter. In other embodiments, the battery can also be arranged in the bottom shell 10 to provide electrical energy to the transmitter 12.

It should be noted that the embodiment of the invention does not limit the shape and position of the second electrical connection area 122. For example, in one embodiment of the invention, the second electrical connection area 122 does not protrude from the surface of the shell 121 of the transmitter, but is flush with the surface of the shell 121 of the transmitter. In another embodiment of the invention, the second electrical connection area 122 is located inside the shell 121 of the transmitter, which will be described in detail below. For example, in another embodiment of the invention, the section of the second electrical connection area is rectangular or circular. In another embodiment of the invention, the conductive part of the second electrical connection area is set on the surface of the connector, or the second electrical connection area 122 itself is the connector. The connector can be inserted into the same elastic member, which will be described in detail below.

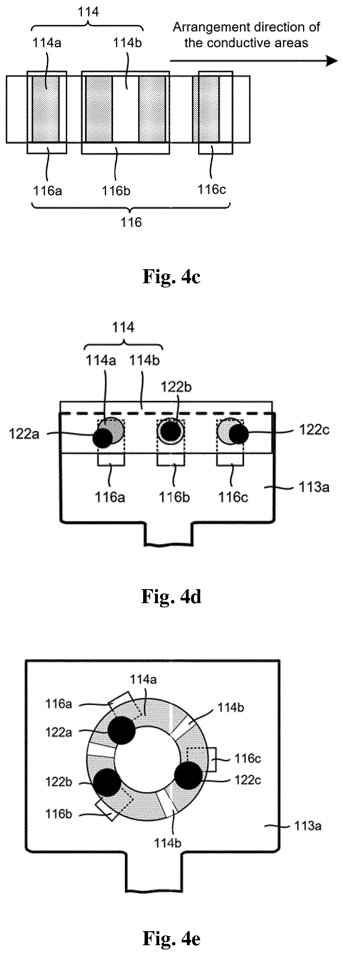

Fig. 4a is the schematic diagram of the top structure of an elastic member, a first electrical connection area and a second electrical connection area in the embodiment of the invention. Fig. 4b is the side view of the elastic member in Fig. 4a. Fig. 4c is the top structure diagram of the elastic member and the first electrical connection area in another embodiment of the invention. Fig. 4d - Fig. 4e shows the top structure of the elastic member, the first electrical connection area and the second electrical connection area in different embodiments of the invention.

First of all, it should be pointed out that the thin dotted line in Fig. 4a represents the contour of the part of the first electrical connection area covered by the elastic member, and the thick dotted line represents the contour of the part of the signal output end covered by the elastic member. The thin dotted lines and thick dotted lines in the following Figs have the same meanings as those here, and will not be repeated below.

The detection device of the embodiment of the invention comprises an elastic member 114. The elastic member 114 contacts the signal output end 113a. Only one elastic member 114 is provided to reduce the number of internal structures of the detection device. In addition, the elastic material will deform after being squeezed, thus playing a locking role. Therefore, the elastic member 114, as a conductive structure or an auxiliary structure as an electrical connection position, can be more closely connected with each other, thereby improving the reliability of electrical connection.

In one embodiment of the invention, the signal output end 113a is arranged at the bottom of the elastic member 114, and the first electrical connection area 116 is indirectly connected with the corresponding second electrical connection area 122. Here, the bottom of the elastic member 114 refers to the part of the elastic member 114 close to the skin.

At this time, the elastic member 114 comprises at least two conductive areas 114a and at least one insulating area 114b. The conductive area 114a and the insulating area 114b play the role of electrical conduction and electrical insulation respectively. The conductive area 114a and the insulating area 114b cannot be separated from each other, that is, the conductive area 114a and the insulating area 114b respectively belong to an integral part of the elastic member 114.

An insulating area 114b is arranged between adjacent conductive areas 114a. Different first electrical connection areas 116 or different second electrical connection areas 122 are electrically connected with different conductive areas 114a, so that any two first electrical connection areas 116 or any two second electrical connection areas 122 are electrically isolated from each other.

Inside the elastic member 114, the conductive area 114a and the insulating area 114b pass through the elastic member 114 in the longitudinal direction, as shown in Fig. 4b. Here, the longitudinal direction refers to the direction from the first electrical connection area 116 to the corresponding second electrical connection area 122, or refers to the direction of the current between the first electrical connection area 116 and the second electrical connection area 122. When the first electrical connection area 116 is electrically connected with the second electrical connection area 122, such a design ensures that the elastic member 114 can only conduct electricity longitudinally and cannot conduct electricity transversely. The elastic member 114 electrically insulates different first electrical connection areas 116 or different second electrical connection areas 122 while electrically connecting the first electrical connection area 116 and the corresponding second electrical connection area 122. One elastic member 114 plays the role of electrical conduction and electrical insulation at the same time, reducing the complexity of the internal structure of the detection device, making the internal structure more compact, and improving the electrical connection reliability of the detection device.

It should be noted that in other embodiments of the invention, the conductive area 114a or the insulating area 114b can also have a certain inclination, or be arranged inside the elastic member 114 in other directions or ways. There is no specific restriction here, as long as the above conditions for electrical conduction and insulation can be met.

Please refer to Fig. 2, Fig. 4a and Fig. 4b. Specifically, in the embodiment of the invention, the elastic member 114 is a cuboid structure. The conductive area 114a and the insulating area 114b are arranged at intervals, and respectively penetrate the elastic member 114. In another embodiment of the invention, different conductive areas 114a are arranged in the same insulating area 114b, that is, surrounded by the same insulating area 114b, as shown in Fig. 4d. In another embodiment of the invention, the top view of the elastic member 114 may be circular, as shown in Fig. 4e. In another embodiment of the invention, the top view of the elastic member 114 can also be circular.

In other embodiments of the invention, the elastic member 114 can also have other shapes, which is not specifically limited here, as long as the conditions for realizing the above functions of the elastic member 114 can be met.

Please continue to refer to Figs. 4a and 4b. When the elastic member 114 is electrically connected with the first electrical connection area 116 and the second electrical connection area 122 respectively, an insulating area 114b is spaced between any two first electrical connection areas 116 connected with the elastic member 114. Specifically, in the embodiment of the invention, the insulation area 114b separated between any two first electrical connection areas 116 comprises a part of an insulation area 114b (as between 116a and 116b in Figs. 4a and 4b), or an insulation area 114b, or more than one insulation area 114b (as between 116c and 116b in Figs. 4a and 4b). Similarly, the insulation area 114b separated between any two second electrical connection areas 122 connected with the elastic member 114 comprises a part of an insulation area 114b, or an insulation area 114b, or more than one insulation area 114b. However, it is obvious that the first electrical connection area and the corresponding second electrical connection area (such as between 116a and 122a, between 116b and 122b, or between 116c and 122c) share the conductive area 114a of the common part, so as to realize the electrical conductivity of both. The conductive area of the common part comprises a part of a conductive area 114a (as between 116c and 122c in Figs. 4a and 4b), or a conductive area 114a, or more than one conductive area 114a.

In combination with Figs. 4a and 4b, it is easy for those skilled in the art to understand that the above-mentioned one insulation area or part of the conductive area, one insulation area or conductive area, and more than one insulation area or conductive area are only the span range of the first electrical connection area or the second electrical connection area in the one-dimensional direction (such as the layout direction of the conductive area.

In other embodiments of the invention, a part of an insulating area or a conductive area, an insulating area or a conductive area, and more than one insulating area or a conductive area can also represent the coverage of the first electrical connection area or the second electrical connection area to the insulating area or the conductive area in a two-dimensional direction (in area), as shown in Fig. 4c. Taking the first electrical connection area as an example, the dotted lines in Fig. 4c represent part of the outline of the first electrical connection area. Obviously, the first electrical connection area 116 can cover a part of an insulating or conductive area, or an insulating or conductive area, or more than one insulating or conductive area.

Obviously, when the number of conductive areas or insulation areas between the above structures is large or the scope is wide, the reliability of electrical connection or electrical insulation between structures will be significantly improved.

In the embodiment of the invention, the materials of the elastic member 114 comprise elastic plastic, elastic rubber, etc. Better electrical contact can be obtained by using the elastic member 114, which also plays a buffering role. When the material of the elastic member 114 is elastic rubber, the elastic member 114 is a conductive rubber strip. One conductive adhesive strip can not only conduct electricity and insulate, but also buffer.

Obviously, when the sensor 113 is a two-electrode system, the number of the first electrical connection area and the second electrical connection area is two. At this time, the elastic member 114 only needs to comprise two conductive areas 114a and an insulating area 114b arranged between the two conductive areas 114a. That is, two pairs of different first electric connection areas and second electric connection areas are electrically connected through different conductive areas 114a to achieve electrical conduction. At the same time, two first electrical connection areas or two second electrical connection areas are separated by insulating areas to achieve electrical insulation.

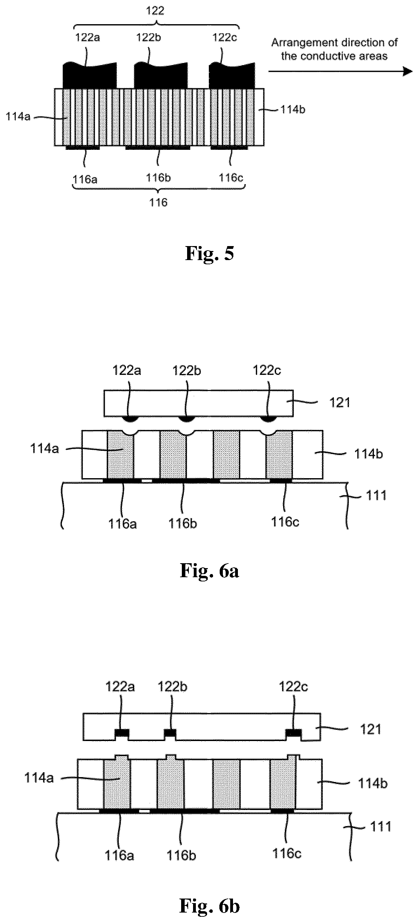

The sensor of other embodiments of the invention can also comprise more electrodes. Therefore, the elastic member 114 comprises more conductive areas and insulating areas which are set at intervals with each other, and the electrical connection mode will be more flexible, as shown in Fig. 5.

It should be noted that in other embodiments of the invention, the sensor comprises at least three electrodes, that is, the signal output end 113a is provided with at least three first electrical connection areas, in which at least two first electrical connection areas are electrically connected with the corresponding second electrical connection area through different conductive areas 114a, and the connection method and principle are consistent with the above. The embodiment of the invention does not limit the connection mode or connection principle of other first electrical connection areas and second electrical connection areas not connected with the elastic member 114. For example, in one embodiment of the invention, the sensor is a three-electrode system, in which only the working electrode and the counter electrode are electrically connected with the second electrical connection area by the corresponding first electrical connection area through the elastic member, while the reference electrode is electrically connected with the transmitter by other means.

Figs. 6a and 6b are the structural diagrams of the electrical connection positions of the second electrical connection area 122 and the elastic member 114 in different embodiments of the invention.

For the convenience of annotation and description, the second electrical connection area 122 and the elastic member 114 in Figs. 6a and 6b will be separated and shown.

As shown in Fig. 6a, in the embodiment of the invention, the second electrical connection area 122 is a convex spherical crown type metal contact. Correspondingly, the elastic member 114 is provided with a concave part (not shown) at the position connected with the protruding metal contact to make the connection closer. At the same time, the connection between the convex part and the concave part also plays a role in fixing the position of the elastic member 114, that is, no matter what external force the detection device is subjected to, the position of the elastic member 114 is always fixed without displacement, so as to ensure that the elastic member 114 performs normal conductive and insulating work.

It should be noted that the elastic member 114 may not be concave. After being squeezed by the protruding metal contact, the elastic member 114 will automatically have a concave part matching with the metal contact to ensure electrical connection or electrical insulation.

As shown in Fig. 6b, in another embodiment of the invention, the second electrical connection area 122 is arranged inside the transmitter 12. At this time, the elastic member 114 is correspondingly provided with a convex part (not shown), which can enter the interior of the transmitter 12 and is electrically connected with the corresponding second electrical connection area 122.

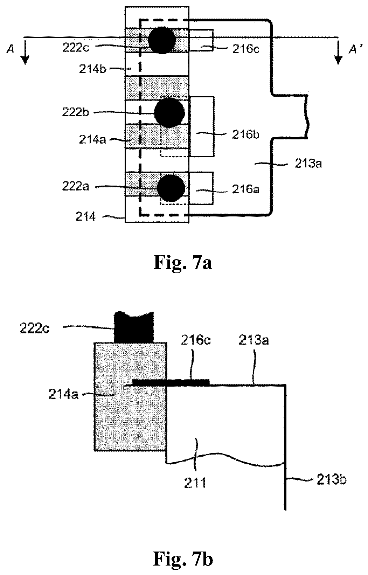

Figs. 7a and 7b are the structural diagrams of the elastic member 214 electrically connected with the first electrical connection area and the second electrical connection area in another embodiment of the invention. Fig. 7a is a top view. Fig. 7b is the sectional view obtained along the section line A-A' in Fig. 7a.

The three second electrical connection areas 222a, 222b and 222c in the embodiment of the invention are respectively indirectly electrically connected with the three first electrical connection areas 216a, 216b and 216c. For the layout of conductive area 214a and insulating area 214b in elastic member 214, please refer to the above.

For details, please refer to Fig. 7b. In the embodiment of the invention, the signal output end 213a is embedded in the elastic member 214. Therefore, the three first electrical connection areas 216a, 216b and 216c are embedded in the elastic member 214. To fix the position of the sensor, the signal output end 213a and the detection end 231b are carried by the sensor base 211.

In the embodiment of the invention, the principle and method of the elastic member 214 to be electrically connected with the first electrical connection area and the second electrical connection area are consistent with those described above.

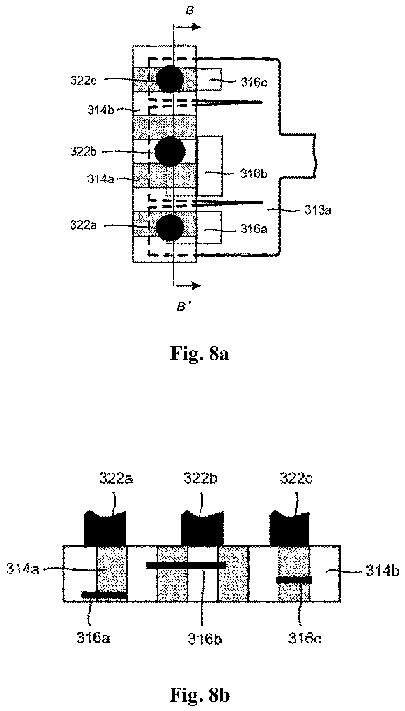

Figs. 8a and 8b are the structural diagrams of the elastic member electrically connected with the first electrical connection area and the second electrical connection area respectively in another embodiment of the invention. Fig. 8a is a top view. Fig. 8b is the sectional view obtained along the section line B-B' in Fig. 8a.

In the embodiment of the invention, different first electrical connection areas are set on different parts of the signal output end 313a, and different parts of the signal output end 313a are independent of each other and do not interfere with each other. Specifically, the three first electrical connection areas are embedded in the conductive area 314a and/or insulating area 314b of the elastic member. As shown in Fig. 8b, in the embodiment of the invention, the height of the embedded positions of the three first electrical connection areas in the elastic member is not completely equal.

In the actual manufacturing process, the thickness of each first electrical connection area will be different. When the transmitter is connected with the sensor, this mutually independent and non-interference first electrical connection area can weaken or eliminate the influence of poor contact caused by the above thickness difference, and improve the reliability of the electrical connection of the three.

Obviously, in other embodiments of the invention, only two of the three first electrical connection areas can be embedded in the elastic member, and the other first electrical connection area is set at the bottom of the elastic member, or the three first electrical connection areas are embedded in the elastic member at the same height. No specific restriction is made here.

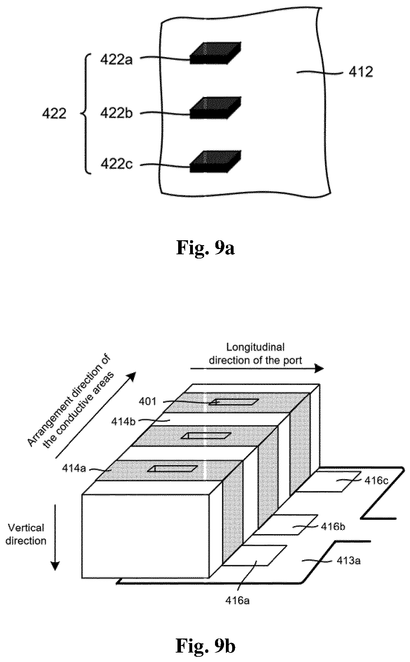

Fig. 9a shows the three-dimensional structure of the second electrical connection area 422 in another embodiment of the invention. Fig. 9b is the schematic diagram of the three-dimensional structure of the elastic member matched with the second electrical connection area 422 in Fig. 9a and the signal output end 413a.

The three second electrical connection areas 422a, 422b and 422c are connectors and protrude out of the transmitter housing 412. The types of connectors are described above. Three jacks 401 are arranged in the elastic member to match with three second electrical connection areas. The three second electrical connection areas can be respectively inserted into the corresponding jack 401.

In the embodiment of the invention, the length direction of the jack 401 is perpendicular to the arrangement direction of the conductive area 414a or the insulating area 414b. In other embodiments of the invention, the two directions can be arbitrarily designed according to requirements. For example, in one embodiment of the invention, the length direction of the jack is parallel to the arrangement direction of the conductive area. For the principle and method of electrical connection, please refer to the previous article.

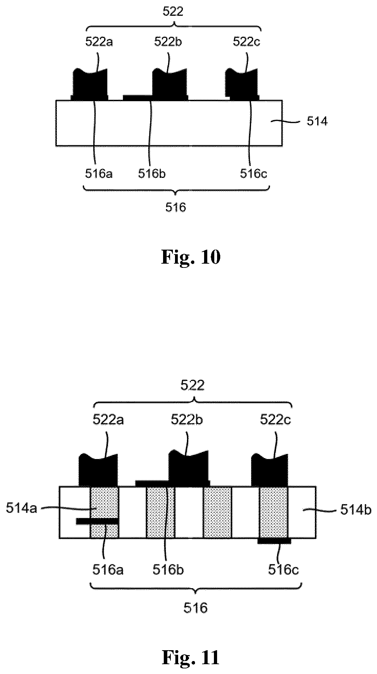

Fig. 10 is a structural diagram of another embodiment of the invention in which the signal output end is arranged on the top of the elastic member 514.

In another embodiment of the invention, the signal output end is set at the top of the elastic member 514, that is, the signal output end is set between the elastic member 514 and the second electrical connection area 522. At this time, the second electrical connection area 522 is directly connected with the corresponding first electrical connection area 516. Therefore, the elastic member 514 can be an ordinary elastic member or an elastic member with a conductive area. Preferably, the second electrical connection area 522 is a projecting metal contact. Since the elastic member 514 is loaded below the first electrical connection area 516, the reliability of the electrical connection between the second electrical connection area 522 and the first electrical connection area 516 is high. Similarly, the shape selection of elastic member 514 can be consistent with the above, and will not be repeated here.

As mentioned earlier, different parts of the signal output end can be independent of each other without interference. Preferably, in another embodiment of the invention, three first electrical connection areas 516 are respectively arranged in different parts of the signal output end. Therefore, three different parts of the signal output end are respectively arranged at different positions of the elastic member. For example, the first electrical connection area 516b is set at the top of the elastic member, the first electrical connection area 516a is embedded in the elastic member 514, and the first electrical connection area 516c is set at the bottom of the elastic member, as shown in Fig. 11. When there are more independent first electrical connection areas 516, the positions of different first electrical connection areas can be selected as required.

In the existing detection device, there are multiple separated conductive parts and/or multiple separated insulating parts between the transmitter and the sensor, and one part can only play one role, which increases the complexity of the internal structure of the detection device. At the same time, the reliability of the electrical connection between the transmitter and the sensor is poor, and the problems of signal interruption and data loss are easy to occur.

To sum up, the invention discloses a highly reliable analyte detection device, wherein the signal output end bends or bends to the bottom surface of the bottom shell, and the signal output end contacts with the same elastic member, which increases the reliability of the electrical connection between the transmitter and the sensor, greatly reduces the possibility of data loss, reduces the number of structures inside the device, and enhances the user experience.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope of the invention. The scope of the invention is limited by the attached claims.

## Claims

1. A high reliability analyte detection device, comprising:
a transmitter (12) which is provided with at least one first clamp part (123) and at least two mutually insulated second electrical connection areas (122);
a bottom shell (10) which is provided with a second clamp part (104) corresponding to the first clamp part (123), wherein the first clamp part (123) and the second clamp part (104) are clamped to each other, and the transmitter (12) is assembled on the bottom shell (10), the bottom shell (10) comprises a fixed part and a forced part, the bottom shell (10) being configured such that during separating the bottom shell and the transmitter (12), the fixed part is configured to be fixed, and the forced part is configured to receive a force in one direction, the bottom shell thereby being in a failure mode, the failure mode comprising one or more combinations of fracture of the bottom plate, fracture of the bottom shell, fracture of the second clamp part, and deformation of the bottom shell, and during separation at least one pair of first and second clamp parts (123, 104) that are clamped to each other are separated from each other, thereby separating the bottom shell (10) from the transmitter (12);
a sensor (113) is assembled on the bottom shell (10), wherein the sensor (113) comprises a signal output end (113a) and a detection end (113b), the signal output end (113a) is provided with at least two first electrical connection areas (116) corresponding to the second electrical connection areas (122) and mutually insulated, wherein the second electrical connection areas (122) are electrically connected with the first electrical connection areas (116), respectively;
an elastic member (114), wherein the signal output end (113a) contacts the elastic member (114); and
a battery, connected to the transmitter (12), for supplying power to the transmitter (12); and
wherein the elastic member (114) comprises at least two conductive areas (114a) and at least one insulating area (114b), the insulating area (114b) is arranged between the two conductive areas (114a) adjacent with each other, the at least two second electrical connection areas (122) are indirectly electrically connected with the first electrical connection areas (116) through the conductive areas (114a), and the second electrical connection areas (122) and the first electrical connection areas (116) are directly electrically connected with the conductive areas (114a).

2. The high reliability analyte detection device of claim 1, wherein the second electrical connection areas (122) are metal contacts.

3. The high reliability analyte detection device of claim 1, wherein the signal output end (113a) is curved or bent towards a bottom surface of the bottom shell (10).

4. The high reliability analyte detection device of claim 3, wherein the conductive areas (114a) and the insulating area (114b) pass through the elastic member (114) in a longitudinal direction, respectively.

5. The high reliability analyte detection device of claim 3, wherein the signal output end (113a) is embedded in the elastic member (114) or arranged at a bottom of the elastic member (114).

6. The high reliability analyte detection device of claim 5, wherein the first electrical connection areas (116) are set on different parts of the signal output end (113a,313a), and the different parts of the signal output end (113a) are independent of each other.

7. The high reliability analyte detection device of claim 6, wherein the signal output end (113a) is embedded in the elastic member (114), and heights of embedded positions of the signal output end (113a) of different parts in the elastic member (114) are not exactly the same.

8. The high reliability analyte detection device of claim 6, wherein the signal output end (113a) of each part is embedded in the elastic member (114), or is arranged at the bottom of the elastic member (114), or is arranged at the top of the elastic member (114).

9. The high reliability analyte detection device of claim 1, wherein a number of the first electrical connection areas (116) and a number of the second electrical connection areas (122) are three, respectively.

10. The high reliability analyte detection device of claim 1, wherein a part of the bottom shell (10) that is equipped with the transmitter (12) is the forced part.

11. The high reliability analyte detection device of claim 1, wherein a side of the bottom shell (10) is provided with a convex part which is outward, and the convex part is the forced part.

12. The high reliability analyte detection device of claim 1, wherein the battery is arranged in the bottom shell (10), and at least one connection hole is arranged in the bottom shell (10), the transmitter (12) is electrically connected with the two poles of the battery through the connection hole, and a battery part is the forced part.

13. The high reliability analyte detection device of claim 12, wherein a sealing ring (130) is arranged around the connection hole to seal the electrical connection position, when the force is applied to the forced part, the sealing ring provides elastic force to promote separation of the bottom shell (10) and the transmitter (12).

## Patentansprüche

1. Eine hochzuverlässige Analytdetektionsvorrichtung, umfassend:
einen Sender (12), der mit mindestens einem ersten Klemmteil (123) und mindestens zwei voneinander isolierten zweiten elektrischen Anschlussbereichen (122) versehen ist;
ein Bodengehäuse (10), das mit einem dem ersten Klemmteil (123) entsprechenden zweiten Klemmteil (104) versehen ist, wobei das erste Klemmteil (123) und das zweite Klemmteil (104) aneinander geklemmt sind und der Sender (12) auf dem Bodengehäuse (10) montiert ist, wobei das Bodengehäuse (10) ein festes Teil und ein kraftaufnehmendes Teil umfasst, wobei das Bodengehäuse (10) so konfiguriert ist, dass beim Trennen des Bodengehäuses und des Senders (12) das feste Teil so konfiguriert ist, dass es fixiert ist, und das kraftaufnehmende Teil so konfiguriert ist, dass es eine Kraft in einer Richtung aufnimmt, wodurch sich das Bodengehäuse in einem Ausfallmodus befindet, wobei der Ausfallmodus eine oder mehrere Kombinationen aus Bruch der Bodenplatte, Bruch des Bodengehäuses, einem Bruch des zweiten Klemmteils und einer Verformung des Bodengehäuses umfasst, und während der Trennung mindestens ein Paar aus einem ersten und einem zweiten Klemmteil (123, 104), die aneinander geklemmt sind, voneinander getrennt werden, wodurch das Bodengehäuse (10) vom Sender (12) getrennt wird;
ein Sensor (113), der an dem Bodengehäuse (10) angebracht ist, wobei der Sensor (113) ein Signalausgangsende (113a) und ein Erfassungsende (113b) umfasst, das Signalausgangsende (113a) mit mindestens zwei ersten elektrischen Verbindungsbereichen (116) versehen ist, die den zweiten elektrischen Verbindungsbereichen (122) entsprechen und gegenseitig isoliert sind, wobei die zweiten elektrischen Verbindungsbereiche (122) jeweils mit den ersten elektrischen Verbindungsbereichen (116) elektrisch verbunden sind;
ein elastisches Element (114), wobei das Signalausgangsende (113a) das elastische Element (114) kontaktiert; und
eine Batterie, die mit dem Sender (12) verbunden ist, um den Sender (12) mit Strom zu versorgen; und
wobei das elastische Element (114) mindestens zwei leitfähige Bereiche (114a) und mindestens einen isolierenden Bereich (114b) umfasst, wobei der isolierende Bereich (114b) zwischen den beiden leitfähigen Bereichen (114a) aneinander angrenzend angeordnet ist,
wobei die mindestens zwei zweiten elektrischen Verbindungsbereiche (122) indirekt elektrisch mit den ersten elektrischen Verbindungsbereichen (116) über die leitfähigen Bereiche (114a) verbunden sind und wobei die zweiten elektrischen Verbindungsbereiche (122) und die ersten elektrischen Verbindungsbereiche (116) mit den leitfähigen Bereichen (114a) direkt elektrisch verbunden sind.

2. Die hochzuverlässige Analytdetektionsvorrichtung nach Anspruch 1, wobei die zweiten elektrischen Verbindungsbereiche (122) Metallkontakte sind.

3. Die hochzuverlässige Analytdetektionsvorrichtung nach Anspruch 1, wobei das Signalausgangsende (113a) in Richtung einer Bodenfläche des Bodengehäuses (10) gekrümmt oder gebogen ist.

4. Die hochzuverlässige Analytdetektionsvorrichtung nach Anspruch 3, wobei die leitfähigen Bereiche (114a) und der isolierende Bereich (114b) jeweils in Längsrichtung durch das elastische Element (114) verlaufen.

5. Die hochzuverlässige Analytdetektionsvorrichtung nach Anspruch 3, wobei das Signalausgangsende (113a) in das elastische Element (114) eingebettet oder an einer Unterseite des elastischen Elements (114) angeordnet ist.

6. Die hochzuverlässige Analytdetektionsvorrichtung nach Anspruch 5, wobei die ersten elektrischen Verbindungsbereiche (116) an verschiedenen Teilen des Signalausgangsendes (113a, 313a) angeordnet sind und die verschiedenen Teile des Signalausgangsendes (113a) voneinander unabhängig sind.

7. Die hochzuverlässige Analytdetektionsvorrichtung nach Anspruch 6, wobei das Signalausgangsende (113a) in das elastische Element (114) eingebettet ist und die Höhen der Einbettungspositionen des Signalausgangsendes (113a) verschiedener Teile in dem elastischen Element (114) nicht genau gleich sind.

8. Die hochzuverlässige Analytdetektionsvorrichtung nach Anspruch 6, wobei das Signalausgangsende (113a) jedes Teils in das elastische Element (114) eingebettet ist oder am Boden des elastischen Elements (114) oder an der Oberseite des elastischen Elements (114) angeordnet ist.

9. Die hochzuverlässige Analytdetektionsvorrichtung nach Anspruch 1, wobei eine Anzahl der ersten elektrischen Verbindungsbereiche (116) und eine Anzahl der zweiten elektrischen Verbindungsbereiche (122) jeweils drei beträgt.

10. Die hochzuverlässige Analytdetektionsvorrichtung nach Anspruch 1, wobei ein Teil des Bodengehäuses (10), der mit dem Sender (12) ausgestattet ist, das kraftaufnehmende Teil ist.

11. Die hochzuverlässige Analytdetektionsvorrichtung nach Anspruch 1, wobei eine Seite des Bodengehäuses (10) mit einem nach außen gerichteten konvexen Teil versehen ist und das konvexe Teil das kraftaufnehmende Teil ist.

12. Die hochzuverlässige Analytdetektionsvorrichtung nach Anspruch 1, wobei die Batterie im Bodengehäuse (10) angeordnet ist und mindestens ein Anschlussloch im Bodengehäuse (10) angeordnet ist, wobei der Sender (12) über das Anschlussloch elektrisch mit den beiden Polen der Batterie verbunden ist und ein Batterieteil das kraftaufnehmende Teil ist.

13. Die hochzuverlässige Analytdetektionsvorrichtung nach Anspruch 12, wobei ein Dichtungsring (130) um das Verbindungsloch herum angeordnet ist, um die elektrische Verbindungsposition abzudichten, wobei der Dichtungsring, wenn die Kraft auf das kraftaufnehmende Teil ausgeübt wird, eine elastische Kraft bereitstellt, um die Trennung des Bodengehäuses (10) und des Senders (12) zu fördern.

## Revendications

1. Dispositif de détection d'analyte de fiabilité élevée, comprenant :
un émetteur (12) qui est pourvu d'au moins une première partie de serrage (123) et d'au moins deux deuxièmes zones de connexion électrique (122) mutuellement isolées;
une coque inférieure (10) qui est pourvue d'une deuxième partie de serrage (104) correspondant à la première partie de serrage (123), dans lequel la première partie de serrage (123) et la deuxième partie de serrage (104) sont serrées l'une à l'autre, et
l'émetteur (12) est assemblé sur la coque inférieure (10);
la coque inférieure (10) comprend une partie fixe et une partie forcée, la coque inférieure (10) étant configurée de telle sorte que lors de la séparation de la coque inférieure et de l'émetteur (12), la partie fixe est configurée pour être fixée, et la partie forcée est configurée pour recevoir une force dans une direction;
la coque inférieure étant ainsi dans un mode de défaillance, le mode de défaillance comprenant une ou plusieurs combinaisons de rupture de la plaque inférieure, de rupture de la coque inférieure, de rupture de la deuxième partie de serrage et de déformation de la coque inférieure;
et lors de la séparation, au moins une paire de première et deuxième parties de serrage (123, 104) qui sont serrées l'une à l'autre sont séparées l'une de l'autre, séparant ainsi la coque inférieure (10) de l'émetteur (12);
un capteur (113) est assemblé sur la coque inférieure (10), dans lequel le capteur (113) comprend une extrémité de sortie de signal (113a) et une extrémité de détection (113b);
l'extrémité de sortie de signal (113a) est pourvue d'au moins deux premières zones de connexion électrique (116) correspondant aux deuxièmes zones de connexion électrique (122) et mutuellement isolées, dans lequel les deuxièmes zones de connexion électrique (122) sont connectées électriquement avec les premières zones de connexion électrique (116), respectivement;
un élément élastique (114), dans lequel l'extrémité de sortie de signal (113a) est en contact avec l'élément élastique (114); et
une batterie, connectée à l'émetteur (12), pour alimenter l'émetteur (12); et
dans lequel l'élément élastique (114) comprend au moins deux zones conductrices (114a) et au moins une zone isolante (114b), la zone isolante (114b) est disposée entre les deux zones conductrices (114a) adjacentes l'une à l'autre;
les au moins deux deuxièmes zones de connexion électrique (122) sont indirectement connectées électriquement avec les premières zones de connexion électrique (116) à travers les zones conductrices (114a), et les deuxièmes zones de connexion électrique (122) et les premières zones de connexion électrique (116) sont directement connectées électriquement avec les zones conductrices (114a).

2. Dispositif de détection d'analyte de fiabilité élevée selon la revendication 1, dans lequel les deuxièmes zones de connexion électrique (122) sont des contacts métalliques.

3. Dispositif de détection d'analyte de fiabilité élevée selon la revendication 1, dans lequel l'extrémité de sortie de signal (113a) est courbée ou pliée vers une surface inférieure de la coque inférieure (10).

4. Dispositif de détection d'analyte de fiabilité élevée selon la revendication 3, dans lequel les zones conductrices (114a) et la zone isolante (114b) traversent l'élément élastique (114) dans une direction longitudinale, respectivement.

5. Dispositif de détection d'analyte de fiabilité élevée selon la revendication 3, dans lequel l'extrémité de sortie de signal (113a) est noyée dans l'élément élastique (114) ou disposée au bas de l'élément élastique (114).

6. Dispositif de détection d'analyte de fiabilité élevée selon la revendication 5, dans lequel les premières zones de connexion électrique (116) sont placées sur différentes parties de l'extrémité de sortie de signal (113a, 313a), et les différentes parties de l'extrémité de sortie de signal (113a) sont indépendantes les unes des autres.

7. Dispositif de détection d'analyte de fiabilité élevée selon la revendication 6, dans lequel l'extrémité de sortie de signal (113a) est noyée dans l'élément élastique (114), et les hauteurs des positions noyées de l'extrémité de sortie de signal (113a) des différentes parties dans l'élément élastique (114) ne sont pas exactement les mêmes.

8. Dispositif de détection d'analyte de fiabilité élevée selon la revendication 6, dans lequel l'extrémité de sortie de signal (113a) de chaque partie est noyée dans l'élément élastique (114), ou est disposée au bas de l'élément élastique (114), ou est disposée au sommet de l'élément élastique (114).

9. Dispositif de détection d'analyte de fiabilité élevée selon la revendication 1, dans lequel un nombre des premières zones de connexion électrique (116) et un nombre des deuxièmes zones de connexion électrique (122) sont de trois, respectivement.

10. Dispositif de détection d'analyte de fiabilité élevée selon la revendication 1, dans lequel une partie de la coque inférieure (10) qui est équipée de l'émetteur (12) est la partie forcée.

11. Dispositif de détection d'analyte de fiabilité élevée selon la revendication 1, dans lequel un côté de la coque inférieure (10) est pourvu d'une partie convexe qui est vers l'extérieur, et la partie convexe est la partie forcée.

12. Dispositif de détection d'analyte de fiabilité élevée selon la revendication 1, dans lequel la batterie est disposée dans la coque inférieure (10), et au moins un trou de connexion est ménagé dans la coque inférieure (10), l'émetteur (12) est connecté électriquement avec les deux pôles de la batterie à travers le trou de connexion, et une partie de batterie est la partie forcée.

13. Dispositif de détection d'analyte de fiabilité élevée selon la revendication 12, dans lequel une bague d'étanchéité (130) est disposée autour du trou de connexion pour sceller la position de connexion électrique, lorsque la force est appliquée à la partie forcée, la bague d'étanchéité fournit une force élastique pour favoriser la séparation de la coque inférieure (10) et de l'émetteur (12).
